(19) Europäisches Patentamt
European Patent Office
Office européen des brevets

(11) **EP 0 586 235 B1**

(12) **EUROPEAN PATENT SPECIFICATION**

(45) Date of publication and mention
of the grant of the patent:
**19.03.1997 Bulletin 1997/12**

(51) Int. Cl.$^6$: **A61K 7/32**

(21) Application number: **93306890.0**

(22) Date of filing: **01.09.1993**

(54) **Antiperspirant actives containing titanium salts and compositions**

Titansalz enthaltende, schweisshemmende Mittel und Zusammensetzung

Antiperspirants contenant des sels de titane et compositions

(84) Designated Contracting States:
**AT BE CH DE DK ES FR GB GR IE IT LI NL PT SE**

(30) Priority: **04.09.1992 GB 9218779**

(43) Date of publication of application:
**09.03.1994 Bulletin 1994/10**

(73) Proprietors:
• **UNILEVER PLC**
**London EC4P 4BQ (GB)**
Designated Contracting States:
**GB IE**
• **UNILEVER N.V.**
**3013 AL Rotterdam (NL)**
Designated Contracting States:
**BE CH DE DK ES FR GR IT LI NL PT SE AT**

(72) Inventors:
• **Hagan, Desmond Bernard**
**South Wirral, Cheshire L66 4TE (GB)**
• **Leng, Francis John**
**Gayton, Wirral L60 8QQ (GB)**

(74) Representative: **Gordon, Naoise Padhraic Edward**
**et al**
**Unilever plc**
**Patent Division**
**Colworth House**
**Sharnbrook**
**Bedford MK44 1LQ (GB)**

(56) References cited:
**FR-A- 2 394 290**          **US-A- 3 090 728**
**US-A- 5 135 741**

## Description

This invention relates to antiperspirant active materials, and antiperspirant compositions for topical application to the human skin.

The antiperspirant market is dominated by products based on aluminium and/or zirconium salts, which are intended to reduce or prevent perspiration at the skin surface, particularly the underarm.

However, doubts as to the safety in use of aluminium salts have stimulated research into alternative antiperspirant actives. From this research, it is known that other metal salts may exhibit an antiperspirant effect. One of these metals is titanium.

For example, US-A-3,090,728 to Carter Products describes the use of certain titanium salts of hydroxy aliphatic carboxylic acids as antiperspirant actives. Salts disclosed in this reference include the mono- and di-acid salts of citrate, gluconate, glycolate and lactate.

However, it is noticeable from this reference that it teaches towards antiperspirant compositions which have a relatively low pH, in the region of pH 2.5 to 5. Above this pH, so the reference says, the composition will not be effective. It is also apparent that compositions according to this teaching, because of the fairly low pH of the composition, may have a tendency to cause skin irritation, particularly in people with delicate skin. Such compositions having such a low pH are also most likely to cause damage to fabrics and clothing.

We have surprisingly found that antiperspirant compositions having good efficacy can be made from titanium salts of hydroxy aliphatic carboxylic acids having a chain length of from C2 to C10, which have a pH greater than 5.

Thus, according to the invention, there is provided an antiperspirant composition for topical application to the human skin, comprising an effective amount of a titanium salt of a hydroxy aliphatic carboxylic acid having from 2 to 10 carbon atoms in its aliphatic chain, the composition having a pH in aqueous solution of greater than 5.

The present invention, and in particular preferred features and embodiments thereof, will now be described in detail.

In the accompanying drawings:

Figure 1 is a schematic diagram of the apparatus used to conduct the in vitro test procedure for compositions according to the invention, as described in the Examples hereinbelow;

Figure 2 is a graph showing flow rate in the apparatus as a function of applied pressure, with the cell detached, which is used as a correction curve in the in vitro test procedure.

Preferably the titanium salt is present in the compositions of the invention at a concentration of from about 0.1 to about 50% by weight of the composition. The amount of the salt required for an effective antiperspirant activity may depend on the product form in which the composition is provided for use.

For example, typical preferred concentrations of the salt in the case of solid sticks, lotions, creams and the like are from about 5 to about 50% by weight of the composition, more preferably from about 10 to about 40% by weight of the composition, most preferably from about 15 to about 25% by weight of the composition. In the case of propellant driven aerosol compositions, for example, the concentration of the salt may typically be lower than the above figures, e.g. preferably in the range of from about 0.1 to about 20% by weight of the total composition, more preferably from about 0.5 to about 15% by weight of the total composition, most preferably from about 1 to about 10% by weight of the total composition.

Preferably, the pH of the composition in aqueous solution is greater than about 5.1, especially greater than about 5.5, particularly from about 5 or 5.5 to about 10.5, preferably from about 6 to about 10, more preferably from about 6 to about 7.

The pH of the composition may readily be measured where the composition is aqueous. However, where the composition is anhydrous, or contains insufficient water to allow a pH reading to be meaningful, the pH for the purposes of this invention may be determined by taking an aqueous solution of the titanium salt in an aqueous composition at the same concentration as it is found in the anhydrous composition, along with any other components of the composition that would affect the pH, such as buffers. In these circumstances, the pH of the composition in the aqueous solution should be greater than about 5, e.g. greater than about 5.5, preferably in the region of about 6 to 10.

Compositions according to the invention have improved mildness to the skin compared with the compositions of US-A-3,090,728, and provide less risk of fabric damage or skin irritation. However, and contrary to the teaching of US-A-3,090,728, we have surprisingly found that such compositions having a pH greater than 5 do in fact exhibit a remarkably good efficacy as antiperspirant compositions.

Preferred hydroxy aliphatic carboxylic acids for use in the invention are those which have a C2 to C10 aliphatic carbon chain carrying from 1 to 6 hydroxyl groups.

Preferred titanium hydroxy aliphatic carboxylic acid salts according to the invention are the mono- and di-acid salts of citrate, gluconate, glycolate, and lactate, especially lactate.

The pH of the compositions according to the invention may conveniently be adjusted with acids or bases, such as hydrogen chloride or ammonia. A particular advantage of systems comprising titanium lactate is that such a system may additionally comprise its own buffer system, wherein the lactate/lactic acid equilibrium in the system adjusts itself as the amount of acid in the composition is varied. Such a buffer system may typically retain the composition at a pH in the range 5.5-8. However it is thought that other preferred salts, such as citrate, may also be capable of generating their own buffer system in the composition.

Thus, a particularly preferred embodiment of the invention involves a composition which has as its antiperspirant active predominantly an ammonium titanium lactate salt. Such a compound is commercially available.

Other Ingredients

The antiperspirant composition according to the invention may comprise other ingredients, depending on the nature and form of the finished product.

Examples of other ingredients which can optionally be present in a composition according to the invention include:

- cosmetically acceptable vehicles, such as straight-chain and branched chain alcohols, for example ethanol, isopropanol, or isobutanol;
- volatile and non-volatile silicones, such as dimethyl cyclosiloxanes, such as DOW CORNING fluids DC 344 and DC 345, or polydimethylsiloxane, having a viscosity in excess of 5 $mm^2s^{-1}$, for example from 50 to 100 $mm^2s^{-1}$, such as DOW CORNING 200 Fluids (standard viscosities 50-1000 $mm^2s^{-1}$);
- deodorants;
- deoperfumes, and deodorant compounds which can also act as antimicrobial agents, such as unsaturated fatty acids, and monoglycerides, for example glycerol monolaurate;
- hydrophobic oils, such as liquid paraffin oils;
- inorganic electrolytes, such as sodium chloride and sodium sulphate
- cationic polymers, such as ABILQUAT 3272 and ABILQUAT 3270, both ex.TH Goldschmidt AG;
- thickeners, such as clays, for example Bentone 38 (trade mark), silicas, for example Aerosol 200 (trade mark), and hydroxypropyl celluloses such Klucel (trade mark);
- skin feel improvers, such as talc and finely divided polyethylene, an example of which is ACUMIST B18;
- gelling agents, such as stearyl alcohol or waxes, for example castor wax;
- emulsifiers, in particular in stick compositions;
- humectants, such as polyols, for example glycerol;
- emollients;
- perfumes;
- preservatives and antioxidants;
- skin benefit agents, such as allantoin;
- colours;
- other cosmetic adjuncts conventionally employed in stick, roll-on lotion, liquid spray, cream, and propellant-driven aerosol antiperspirant products.

The ingredients other than the antiperspirant active can conveniently form the balance of the composition, and accordingly may form up to 95% by weight of the total composition, preferably from 60 to 90% by weight of the total composition, most preferably 75-85% by weight of the composition.

Product Form

The composition according to the invention may take the form of a liquid or solid product, each of which is suited to, or adapted for, topical application to human skin. One convenient form of the composition according to the invention is a solid stick, usually contained in a suitable holder or dispenser to enable it to be applied to the area of the skin, particularly the underarm, where control of perspiration and deodorancy is required.

Another form of the composition of the invention is a lotion suitable for inclusion in a roll-on dispenser, fitted with a ball valve, to enable the product to be rolled on to the skin in a manner which is conventional in the art. A further example of a composition according to the invention is the liquid composition for dispensing via a finger-operated pump spray or a hand-operated squeeze spray to provide for delivery to the skin of a finely divided spray or aerosol, without the use of propellant gases to deliver it.

Alternatively, a composition according to the invention can take the form of liquid, containing suspended particulate solids, which is suited to, or adapted for, topical application to human skin from an aerosol container by use of a suitable propellant, examples of which are well known in the art. The aerosol container can then be used to dispense the composition as a spray to enable it to be applied to the area of the skin, particularly the underarm, where control of perspiration and deodorancy is required.

The composition according to the invention can also take the form of a cream, suited to, or adapted for, topical application to the human skin.

In a preferred embodiment of the invention, the antiperspirant active is present in the composition as a finely divided powder suspended in a substantially anhydrous medium in which the antiperspirant active is highly insoluble. By "substantially anhydrous medium" in this context is meant one that contains less than 1% by weight water. The anhydrous medium is conveniently a liquid medium, and is preferably a substantially anhydrous alcohol comprising ethanol, isopropanol, or mixtures thereof. In a lotion type formulation, the anhydrous medium will conveniently make up from 50-80% by weight of the composition. composition.

Use of the composition

The invention provides for the use of an antiperspirant composition in accordance with the invention in perspiration control, following topical application to the human skin.

A particularly preferred composition according to the invention is an antiperspirant composition containing an antiperspirant active according to the invention, and a hydrophobic clay, especially a Bentone (trade mark) clay, most-especially Bentone 38. It has been found that formulations containing Bentone clays have superior properties to similar compositions not containing Bentone in terms of improved efficacy. The Bentone clay may be present in the formulation at a concentration of from 5-20%, more preferably 8-15% by weight of the total composition.

A further preferred composition according to the invention is one which comprises a surfactant which strongly interacts with the skin, thereby causing improved adhesion of the antiperspirant active to the skin. Such surfactants may comprise, for example, cationic surfactants, or alphahydroxy acids. Preferably, such additives may be present in the composition at a concentration of from 0.1 to 2% by weight of the total composition.

Examples

The invention will now be further described by way of example only. The following compositions were prepared, using standard techniques known in the art. For the formulations not containing Bentone, it was sufficient simply to mix together the components of the mixture, and make the composition to the appropriate amount with a solvent such as ethanol. However, for the formulations containing Bentone, it was necessary to shear the Bentone into an aliquot of the ethanol, at a high rate of shear (e.g. at approximately 75% of the maximum speed of an "Ultraturrax" mixer ) for at least 5 minutes at a temperature of at least 45°C, before cooling the mixture and adding the remainder of the components of the composition.

Example 1

| Composition 1 (% w/w) | |
|---|---|
| Component | 1 |
| Tyzor LA (1) | 20 % |
| Hydrogen chloride (as aqueous hydrochloric acid) (2) | 0.6% |
| Perfume (3) | 1.0% |
| Klucel M (4) | 0.8% |
| R R Ethanol | 51.6% |
| Distilled water | to 100 % |

(1) Tyzor LA is ammonium titanium lactate, ex. Du Pont, supplied as a 50 % w/w aqueous solution.
(2) Included to reduce the pH from around 10 to 7.8.
(3) (HD400, ex Quest).
(4) ex Aqualon.

Composition 1 was formulated as a roll-on lotion.

For comparison, an **in vivo** test procedure used to evaluate the efficacy of Composition 1 was also used on Composition 2, the commonly available "Sure" (trade mark) roll-on antiperspirant. This composition is available in the UK and is considered to be the most efficacious aqueous ethanolic zirconium aluminium chlorohydrate product commercially available, and contains 15% by weight of the zirconium/aluminium active.

## Example 2

### **In vivo** test procedure

Each of the compositions was tested in a standard hot-room assessment procedure. In this, human volunteers are subjected to thermal stress and gravimetric determination of the perspiration produced under the thermal stress, and this is summarised as follows:

Subjects: Panels of around 66 women who use no antiperspirants for the 14 days before the test.
Hot room: Temperatures 40°C ± 2°C; relative humidity 40% ± 5%.

Test Design: Subjects attended daily for 3 consecutive days. They received one treatment with the products on each of the three days. After product application on the third day, the panellist was subjected to a hot-room sitting in which sweat was collected.

Products: When testing two products, one being designated the test product and the other the control, the panel is divided into two equal groups. One group receives the test treatment on the left axilla and the control treatment on the right, while the second group receives them the other way round.

Product Application: The operator conducting the test applies the test product in a standard manner, so as to deposit an appropriate quantity of product on each axilla. For a stick or roll-on product this will be on average about 300 mg of product to each axilla, whereas for an aerosol product approximately 1-1.5 grams of product is dispensed.

Sweat Collection: Absorbent "Raytex" cotton pads are used to collect the sweat. On entering the hot room, each panellist has the test product applied to the axilla, and then is subjected to a 40-minute 'warm-up' period, during which no sweat is collected. After this, sweat is then collected for a 20-minute period and the sweat weight determined.

Analysis of Data: The statistical treatment includes an analysis of variance which allows for effects due to the different degree of sweating from the different axillae of a given panellist. The efficacy is calculated from the geometric mean weight of sweat collected from the axillae treated with each product using the formula:

$$\% \text{ sweat reduction} = 100 \frac{(C-T)}{C}$$

where C is the geometric mean sweat weight from the axillae treated with the control product and T is the geometric mean sweat weight from the axillae treated with the test product where a correction has been made for the side effect.

Significance is calculated by applying Student's t-test to the logarithmically transformed weights.

Results

| Composition | % sweat reduction |
|---|---|
| 1 | 25 |
| 2 | 32 |

### **In vitro** test procedure

Compositions according to the invention were also subjected to an **in vitro** test method to investigate their efficacy as antiperspirant actives. The formulation of the compositions used for this test are given in Table 1 below.

The following describes the apparatus and test protocol used. The apparatus and test protocol are based on the apparatus and method described by H.H. Reller & W.L. Luedders, "Pharmacologic and toxicological effects of topically applied agents on the eccrine sweat glands", Mod. Toxicol. 4: 1-54 (1977).

### Apparatus Design

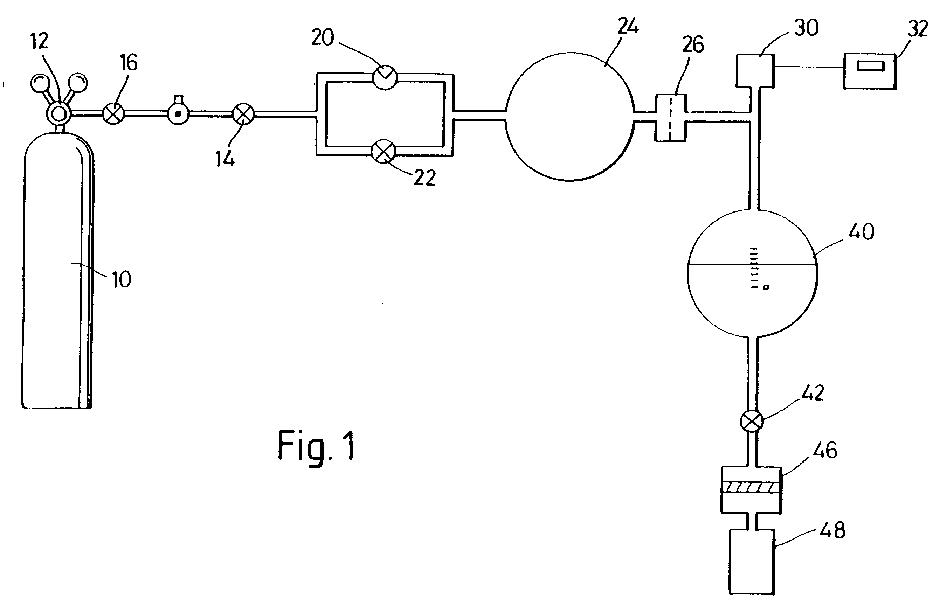

The apparatus used to approximate the degree of pore blocking that would be provided by antiperspirant compositions on the skin surface is shown schematically in Figure 1 of the accompanying drawings. The apparatus comprises four major elements, namely:

(a) the pressure control unit,
(b) the sweat reservoir,
(c) the cell, and
(d) the detection and measurement system.

(a) Pressure Control Unit (10-32)

A white spot nitrogen cylinder 10 and gas regulator 12 are connected to an on/off isolation valve 14 and pressure release safety valve 16. Stainless tubing and Swagelock couplings are used for subsequent connections. The primary pressure source is followed by a parallel arrangement of needle 20 and on/off 22 valves, a gas ballast reservoir 24 and a 0.5 micron particle filter 26. The unit is terminated via a 0 to 3 Bar pressure transducer 30, with accompanying three and a half digit meter 32.

The pressure unit delivers a controlled pressure, which may be stepped or ramped as a function of time. In these experiments, which were to evaluate flow rates, the stepped mode of the apparatus was used.

The in-line particle filter 26 eliminates contamination of the sweat reservoir 40.

(b) "Sweat " Reservoir (40)

This is a laboratory-grade glass reservoir of one litre capacity. Connection to the preceding pressure unit is via a glass to metal seal and Swagelock coupling. Connection to the subsequent cell 46 is via a rapid action on/of valve 42 and Tygon tubing, terminated in the male portion of a Luer-Lok fitting. The reservoir is easily removed for cleaning. For experiments evaluating flow rates with lipid test substances, the "sweat" may be distilled water. However, when this test method is used to evaluate ionic antiperspirant actives, the "sweat" should be a 0.2-0.3% saline solution.

Both the pressure control and reservoir units are enclosed in an aluminium box, for safety reasons. Normal operation involves a maximum pressure of less than one atmosphere above ambient.

(c) Cell (46)

A 5 $\mu$m Millipore SM filter was held in a stainless steel Millipore holder with a Luer-Lok fitting. This particular filter has a simple well-defined structure, and is appropriate for the materials tested.

(d) Detection System (48)

Liquid was collected in a measuring cylinder in the flow studies, with the rate of fluid collection being measured.

Experimental

Millipore filters are immersed in double-distilled water, at 60°C, for several hours. Occasional agitation and several water changes ensure thorough cleaning. The container is covered to keep particulate contamination to a minimum.

The "sweat" reservoir is filled to a predetermined mark with filtered, double-distilled water and pressurised to 0.2 atmospheres above atmospheric pressure via the pressure unit.

The filter is transferred to the cell, flooded with distilled water and attached to the reservoir, ensuring that all air bubbles have been expelled. A flow experiment is commenced by opening the rapid action valve between the cell and reservoir.

The average of three ten-second collections gives the unblocked filter flow rate. The filter is then impregnated with a test solution of antiperspirant active by immersion for three minutes.

The filter is removed, gently shaken, or blotted with a solution-impregnated filter paper, and introduced into a "sweat" solution to gel the entrapped test solution.

The average of three further flow experiments gives the blocked filter flow rate.

The fractional increase in filter blockage equals the fractional flow rate reduction (FFRR), where

$$FFRR = 1 - \text{Blocked flow rate (Ifb)/Unblocked flow rate (Ifu)},$$

for a constant pressure drop(PD) across the filter.

However, it is the total applied pressure (P) that is fixed in the present experiments and allowance must be made for the pressure drop in the apparatus preceding the cell; this pressure drop varies with flow rate.

The corrected formula is

$$FFRR = 1 - (Pfu/Pfb).(Ifb/Ifu)$$

where Pfb is the pressure drop in the apparatus preceding the filter with the filter blocked;

Pfu is the pressure drop in the apparatus preceding the filter with the filter unblocked;

Ifb is the flow rate through the blocked filter; and

Ifu is the flow rate through the unblocked filter.

The portion of the calculation which contains Pfu and Pfb represents a correction factor for the pressure drop which occurs in the apparatus before the filter, which is dependent on flow rate.

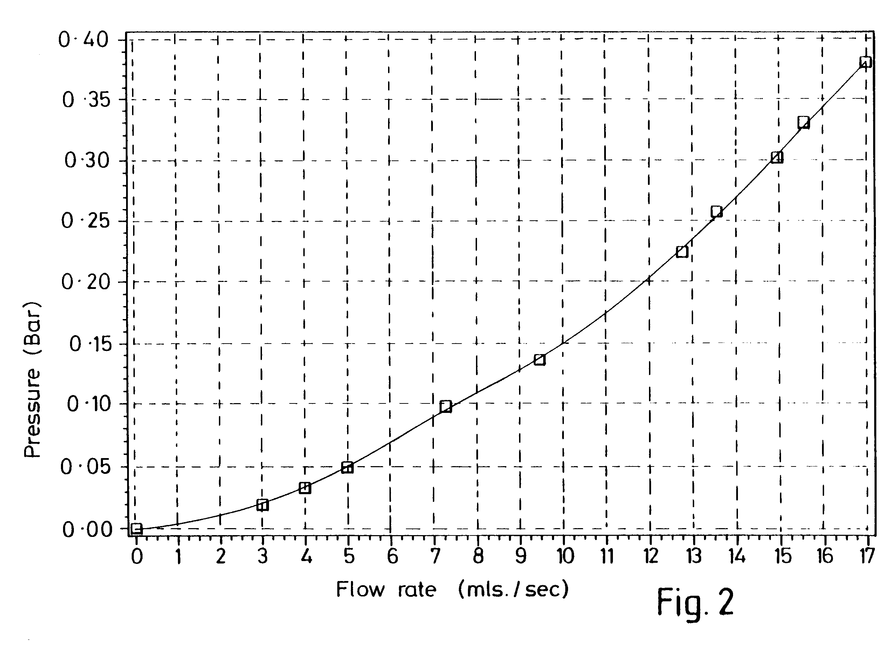

Figure 2 of the accompanying drawings shows the apparatus flow rate as a function of applied pressure, with the cell detached. Pfu and Pfb are read from curve, at the unblocked and blocked experimental flow rates, respectively.

**TABLE 1**

Composition

| Component | 3 | 4 | 5 | 6 | 7 | 8 | 9 |
|---|---|---|---|---|---|---|---|
| Tyzor LA (1) | 1.5 | 3.0 | 6.0 | 6.0 | 6.0 | 6.0 | 10.0 |
| Hydrogen Chloride (as aqueous HCl) | - | - | - | 0.4 | 0.27 | 0.54 | 0.54 |
| Tilcom TL (2) | - | - | - | - | - | - | - |
| NaOH (3) | - | - | - | - | - | - | - |
| Chloracel (4) | - | - | - | - | - | - | - |
| ACH | - | - | - | - | - | - | - |
| Aqueous ethanol (5) | to 100% | - | - | - | - | - | - |

**TABLE 1** (Cont...)

| Component | 10 | 11 | 12 | 13 | 14 |
|---|---|---|---|---|---|
| Tyzor LA (1) | – | – | – | – | – |
| Hydrogen Chloride (as aqueous HCl) | – | – | – | – | – |
| Tilcom TL (2) | 0.3 | 0.3 | 0.3 | – | – |
| NaOH (3) | – | 1.1 | 2.2 | – | – |
| Chloracel (4) | – | – | – | 1.5 | 3.0 |
| ACH | – | – | – | – | – |
| Aqueous ethanol (5) | --- | --- | --- | --- | --- |

(1) Ammonium titanium dilactate, supplied as a 50% w/w aqueous solution, ex Du Pont.
(2) Dilactic acid chelated titanium hydroxide.
(3) Added as a O.1.M. solution.
(4) Sodium Aluminium Chlorohydroxy lactate (ex Reheis).
(5) Ethanol: distilled water ratio 2:1.

EP 0 586 235 B1

**RESULTS**

| Formulation | % in vitro pore blocking |
|---|---|
| 3 | $13 \pm 2$ |
| 4 | $10 \pm 4$ |
| 5 | $10 \pm 4$ |
| 6 | $10 \pm 4$ |
| 7 | $12 \pm 4$ |
| 8 | $8 \pm 2$ |
| 9 | $10 \pm 2$ |
| 10 | $12 \pm 2$ |
| 11 | $11 \pm 2$ |
| 12 | $10 \pm 2$ |
| 13 | $2.0 \pm 0.2$ |
| 14 | $2.0 \pm 0.2$ |

The results clearly show that at pH's characteristic of these compositions of the invention tested, compositions containing Tyzor LA and Tilcom TL are superior in their pore blocking ability over Chloracel, a conventional aluminium-based antiperspirant active.

Example 3

The following compositions were made up by conventional techniques.

Table 2

| Composition (% w/w) | | | |
|---|---|---|---|
| **Component** | 15 | 16 | 17 |
| Ammonium titanium dilactate (1) | 20 | 20 | 20 |
| Klucel M (2) | 0.75 | 0.75 | 0.75 |
| Perfume | 1.0 | 1.0 | 1.0 |
| HCl soln | as required* | as required* | as required* |
| Ethanol/water (3) | to 100% | to 100% | to 100% |

(1) Supplied as a 50% w/w aqueous solution, ex Du Pont.
(2) Hydroxy propyl cellulose, ex. Hercules.
(3) Added to ensure the final ratio of Ethanol/water in the composition was 3:1, including water in the ammonium titanium dilactate solution.
*The compositions 15 , 16 and 17 were adjusted using HCl such their final pH's were 3.5, 6.0 and 8.0 respectively.

When tested on a hot room panel according to the protocol described in Example 2 above, there was found to be no experimentally significant variation in antiperspirant efficacy between the three compositions.

Example 4

The following compositions, which were prepared by conventional techniques, illustrate further examples of antiperspirant compositions in accordance with the invention.

Composition 18: Alcoholic Roll-On Antiperspirant

| Ingredient | % wt. |
|---|---|
| Titanium Dilactate (50% soln., Tilcom TL ex. Tioxide) | 20.00 |
| Hydroxypropylcellulose | 0.75 |
| Ethanol | 64.25 |
| Ammonia Solution (25% wt.) [to pH 6.0] | 4.20 |
| Fragrance | 1.00 |
| Distilled Water | to 100.0% |

Composition 19: Alcoholic Roll-On Antiperspirant

| Ingredient | % wt. |
|---|---|
| Titanium Diglycollate (50% soln.) | 30.00 |
| Hydroxyethylcellulose | 0.60 |
| Ethanol | 35.00 |
| Propylene Glycol | 2.00 |
| Sodium Hydroxide Solution (20% wt.) | to pH 5.5 |
| Fragrance | 1.00 |
| Distilled Water | to 100.0% |

Composition 20: Non-Alcoholic Roll-On Antiperspirant

| Ingredient | % wt. |
|---|---|
| Sodium Titanium Citrate (Tilcom STC ex. Tioxide) | 10.00 |
| POE-21 Stearyl Ether | 3.00 |
| POE-2 Stearyl Alcohol | 1.00 |
| POP-15 Stearyl Alcohol | 3.00 |
| Disodium EDTA | 0.10 |
| BHT | 0.05 |
| Fragrance | 1.00 |
| Sodium Hydroxide Solution (20%) | to pH 5.1 |
| Distilled Water | to 100.0% |

Composition 21: Dry Roll-On Antiperspirant

| Ingredient | % wt. |
|---|---|
| Diammonium Titanium 2-Hydroxyhexanaoate | 15.00 |
| Decamethylcyclopentasiloxane | 75.00 |
| Dimethicone | 5.00 |
| Modified Hydrophobic Clay (Bentone 38) | 3.00 |
| Ethanol | 1.00 |
| Fragrance | 1.00 |

Composition 22: Non-Greasy Roll-On Antiperspirant

| Ingredient | % wt. |
|---|---|
| Titanium $\alpha$-Hydroxy Butyrate | 20.00 |
| Isostearyl Benzoate | 10.00 |
| Potassium Hydroxide Solution (50% wt.) | to pH 7.0 |
| Cyclomethicone | 23.00 |
| Fragrance | 1.00 |
| Distilled Water | to 100.0% |

Composition 23: Roll-On Antiperspirant

| Ingredient | % wt. |
|---|---|
| Titanium Glucuronate | 18.00 |
| Magnesium Aluminium Silicate | 1.00 |
| Glycerol Monostearate (acid-stable) | 8.00 |
| Cyclomethicone | 2.00 |
| Triethanolamine | to pH 5.1 |
| Fragrance | 1.25 |
| Distilled Water | to 100.0% |

Composition 24: Emulsion Roll-On Antiperspirant

| Ingredient | % wt. |
|---|---|
| Titanium Lactate | 25.00 |
| Cetostearyl Alcohol | 6.00 |
| Phenoxyethanol | 0.20 |
| IPM | 0.50 |
| Glycerol Monostearate (acid-stable) | 7.50 |
| Ammonia Solution (25% wt.) (to pH 6.0) | 10.50 |
| Titanium Dioxide | 0.20 |
| Octyl Methoxycinnamate | 0.20 |
| Fragrance | 1.00 |
| Distilled Water | to 100.0% |

Composition 25: Alcoholic Gel Roll-On Antiperspirant

| Ingredient | % wt. |
|---|---|
| Titanium Malate | 17.00 |
| Sorbitol (70% soln.) | 8.00 |
| Butane-1, 3-diol | 10.00 |
| PEG 400 | 9.00 |
| Volatile Silicone | 20.00 |
| Fragrance | 1.00 |
| Potassium Hydroxide Solution (25% wt.) | to pH 7.5 |
| Distilled Water | to 100.0% |

Composition 26: Carbopol Gel Roll-On Antiperspirant

| Ingredient | % wt. |
|---|---|
| Titanium Dilactate (50% soln., Tilcom TL ex. Tioxide) | 24.00 |
| Carbopol 1342 | 1.00 |
| Glycerol | 10.20 |
| Fragrance | 1.00 |
| Triethanolamine | to pH 7.8 |
| Distilled Water | to 100.0% |

Composition 27: Aerosol Antiperspirant

| Ingredient | % wt. |
|---|---|
| Sodium Titanium Dilactate | 16.00 |
| Bentone 38 | 4.00 |
| PPG-14 Butyl Ether | 6.00 |
| Fragrance | 4.00 |
| Cyclomethicone | 70.00 |

This base composition was used as 25% by weight of the total formulation with 75% hydrocarbon propellant.

Composition 28: Aerosol Antiperspirant

| Ingredient | % wt. |
|---|---|
| Ammonium Titanium Di(a-Hydroxyisobutyrate) | 45.00 |
| Fumed Silica | 8.00 |
| IPM | 37.00 |
| Fragrance | 10.00 |

This base composition was used as 23% by weight of the total formulation with 77% hydrocarbon propellant.

Composition 29: High Efficacy Aerosol

| Ingredient | % wt. |
|---|---|
| Solid Diammonium Titanium Lactate | 6.00 |
| Bentone 38 (Hydrophobic Clay Derivative) | 2.00 |
| Ethanol | 9.50 |
| Urea | 0.50 |
| Cyclomethicone Pentamer Blend | 1.00 |
| Perfume | 1.00 |
| Pentane | 37.00 |
| Hydrocarbon Propellant (CAP 30) | 43.00 |

Composition 30: Dry Suspensoid Stick Antiperspirant

| Ingredient | % wt. |
|---|---|
| Sodium Titanium Citrate | 20.00 |
| Stearyl Alcohol | 23.00 |
| Hydrogenated Castor Oil | 4.00 |
| Glycerol Monostearate | 1.00 |
| PPG-14 Butyl Ether | 2.00 |
| Magnesium Aluminium Silicate | 1.00 |
| Perfume | 1.00 |
| Cyclomethicone Pentamer Blend | 47.00 |

Composition 31: Dry Suspensoid Stick Antiperspirant

| Ingredient | % wt. |
|---|---|
| Ammonium Titanium Diethyleneglycol Dilactate | 30.00 |
| Stearyl Alcohol | 20.00 |
| Hydrogenated Castor Oil | 4.00 |
| BHT | 0.05 |
| PEG-150 Distearate | 1.00 |
| Magnesium Aluminium Silicate | 3.20 |
| Perfume | 1.50 |
| Cyclomethicone Pentamer/Tetramer Blend | 40.25 |

Composition 32: Unfragranced Dry Suspensoid Stick Antiperspirant

| Ingredient | % wt. |
|---|---|
| Potassium Titanium Glucuronate | 35.00 |
| Stearyl Alcohol | 19.00 |
| Hydrogenated Castor Oil | 4.00 |
| Glycerol Tribehenate | 2.00 |
| PEG-8 Distearate | 1.00 |
| Talc | 4.00 |
| Cyclomethicone Pentamer/Tetramer Blend | 47.00 |

Composition 33: Dry Suspensoid Stick Antiperspirant

| Ingredient | % wt. |
|---|---|
| Triethanolammonium Titanium 2-Hydroxy Octanoate | 18.00 |
| Stearyl Alcohol | 22.00 |
| PPG-2 Myristyl Ether Propionate | 45.00 |
| Glycerol Tribehenate | 2.00 |
| PPG-15 Stearyl Ether | 6.00 |
| Silicone Dioxide | 1.00 |
| POE-5 Oleyl Ether | 1.00 |
| C18-C36 Triglycerides | 4.00 |
| Perfume | 1.00 |

Composition 34: Moisturising Gel Antiperspirant

| Ingredient | % wt. |
|---|---|
| Diammonium Titanium Lactate (Tyzor LA ex. Du Pont) | 20.00 |
| Propylene Glycol | 60.00 |
| Sodium Stearoyl Lactylate | 15.00 |
| Water | 5.00 |

Composition 35: Stick

| Ingredient | % wt. |
|---|---|
| Monoammonium Titanium (2-Amino-2-methyl-1-propanol) Lactate Diethyleneglycol | 10.00 |
| Dibenzylidene Sorbitol | 3.00 |
| Ethanol | 41.00 |
| Propylene Glycol | 30.00 |
| Zinc Acetate | 0.50 |
| Stearic Acid | 0.50 |
| Cyclomethicone Pentamer Blend | 10.00 |
| POP-15 Stearyl Alcohol | 5.00 |

Composition 36: High Efficacy Stick

| Ingredient | % wt. |
|---|---|
| Ammonium Titanium Monocitrate | 13.00 |
| Bentone 38 | 6.00 |
| Behenyl Alcohol | 13.00 |
| Propylene Glycol | 20.00 |
| Dipropyleneglycol Methyl Ether | 7.00 |
| Talc | 4.00 |
| Cyclomethicone Tetramer Blend | 4.00 |
| Perfume | 1.00 |
| Ethanol | 22.00 |

Composition 37: Unfragranced Pump Antiperspirant

| Ingredient | % wt. |
|---|---|
| Potassium Titanium Dicitrate | 15.00 |
| Ethanol | 72.00 |
| Hydrogenated Castor Oil | 1.00 |
| Isopropyl Myristate | 6.00 |
| Propylene Glycol | 6.00 |

Composition 38: Pump Antiperspirant

| Ingredient | % wt. |
|---|---|
| Ammonium Titanium 2-Hydroxy Decanoate | 20.00 |
| Ethanol | 59.50 |
| Perfume | 0.50 |
| POP-15 Stearyl Alcohol | 2.00 |
| Stearic Acid | 2.00 |
| Cyclomethicone Tetramer/Pentamer Blend | 16.00 |

Composition 39: Pump Antiperspirant

| Ingredient | % wt. |
| --- | --- |
| Ammonium Titanium Lactate | 10.00 |
| Ethanol | 80.00 |
| Perfume | 1.00 |
| Stearic Acid | 3.00 |
| Propylene Glycol | 5.00 |
| Isopropyl Myristate | 1.00 |

**Claims**

1. An antiperspirant composition suitable for topical application to the human skin, comprising an effective amount of a titanium salt of a hydroxy aliphatic carboxylic acid having a chain length of from $C_2$ to $C_{10}$, the composition having a pH in aqueous solution of greater than 5.

2. An antiperspirant composition according to claim 1, wherein the composition comprises from 0.1-50% by weight of the titanium salt.

3. An antiperspirant composition according to claim 2, wherein the composition comprises from 10 to 40% by weight of the titanium salt.

4. An antiperspirant composition according to claim 2, wherein the composition comprises from 1 to 10% by weight of the titanium salt.

5. An antiperspirant composition according to any preceding claim, wherein the pH of the composition in aqueous solution is greater than 5.5.

6. An antiperspirant composition according to claim 5, wherein the pH of the composition in aqueous solution is in the range 6 to 10.

7. An antiperspirant composition according to claim 6, wherein the pH of the composition in aqueous solution is in the range 6 to 7.

8. An antiperspirant composition according to any preceding claim, wherein the titanium salt is a mono- or di-acid salt of citrate, gluconate, glycolate or lactate.

9. An antiperspirant composition according to claim 8, wherein the antiperspirant active is an ammonium titanium lactate salt.

10. An antiperspirant composition according to any preceding claim, which is substantially anhydrous and additionally comprises ethanol, isopropanol or a mixture thereof.

11. An antiperspirant composition according to claim 10, wherein the isopropanol, ethanol or mixture thereof is present in the composition at a level of from 50-80% by weight.

12. An antiperspirant composition according to any preceding claim, additionally comprising a hydrophobic clay.

13. An antiperspirant composition according to claim 12, wherein the hydrophobic clay is present in the composition at a level of from 5 to 20% by weight.

14. An antiperspirant composition according to any preceding claim, additionally comprising a volatile or a non-volatile silicone.

15. An antiperspirant composition according to any preceding claim, additionally comprising from 0.1 to 2% by weight of a surfactant.

16. An antiperspirant composition according to any preceding claim, which is in the form of a solid stick, lotion, cream or aerosol spray.

17. A non-therapeutic method of preventing or limiting perspiration of the human skin, comprising topically applying thereto an antiperspirant composition according to any one of claims 1 to 16.

**Patentansprüche**

1. Antischweißzusammensetzung mit Eignung zur topischen Applikation auf menschliche Haut, die eine wirksame Menge eines Titansalzes einer hydroxyaliphatischen Carbonsäure mit einer Kettenlänge von 2 bis 10 Kohlenstoffatomen umfaßt und einen pH-Wert in wäßriger Lösung von mehr als 5 aufweist.

2. Antischweißzusammensetzung nach Anspruch 1, wobei die Zusammensetzung 0,1-50 Gew.-% des Titansalzes umfaßt.

3. Antischweißzusammensetzung nach Anspruch 2, wobei die Zusammensetzung 10-40 Gew.-% des Titansalzes umfaßt.

4. Antischweißzusammensetzung nach Anspruch 2, wobei die Zusammensetzung 1-10 Gew.-% des Titansalzes umfaßt.

5. Antischweißzusammensetzung nach einem der vorhergehenden Ansprüche, wobei der pH-Wert der Zusammensetzung in wäßriger Lösung mehr als 5,5 beträgt.

6. Antischweißzusammensetzung nach Anspruch 5, wobei der pH-Wert der Zusammensetzung in wäßriger Lösung in einem Bereich von 6 bis 10 liegt.

7. Antischweißzusammensetzung nach Anspruch 6, wobei der pH-Wert der Zusammensetzung in wäßriger Lösung in einem Bereich von 6 bis 7 liegt.

8. Antischweißzusammensetzung nach einem der vorhergehenden Ansprüche, wobei das Titansalz ein Mono- oder Disäuresalz von Citrat, Gluconat, Glycolat oder Lactat ist.

9. Antischweißzusammensetzung nach Anspruch 8, wobei der Antischweißwirkstoff ein Ammoniumtitanlactatsalz ist.

10. Antischweißzusammensetzung nach einem der vorhergehenden Ansprüche, die im wesentlichen wasserfrei ist und darüber hinaus Ethanol, Isopropanol oder ein Gemisch hiervon umfaßt.

11. Antischweißzusammensetzung nach Anspruch 10, wobei das Isopropanol, Ethanol oder Gemisch hiervon in der Zusammensetzung in einer Menge von 50 bis 80 Gew.-% vorhanden ist.

12. Antischweißzusammensetzung nach einem der vorhergehenden Ansprüche, die darüber hinaus einen hydrophoben Ton umfaßt.

13. Antischweißzusammensetzung nach Anspruch 12, wobei der hydrophobe Ton in der Zusammensetzung in einer Menge von 5 bis 20 Gew.-% vorhanden ist.

14. Antischweißzusammensetzung nach einem der vorhergehenden Ansprüche, die darüber hinaus ein flüchtiges oder nichtflüchtiges Silicon umfaßt.

15. Antischweißzusammensetzung nach einem der vorhergehenden Ansprüche, die darüber hinaus 0,1 bis 2 Gew.-% eines grenzflächenaktiven Mittels umfaßt.

16. Antischweißzusammensetzung nach einem der vorhergehenden Ansprüche in Form eines festen Stifts, einer Lotion, einer Creme oder eines Aerosolsprays.

**17.** Nichttherapeutisches Verfahren zum Verhindern oder Einschränken der Schweißbildung der menschlichen Haut durch topisches Applizieren einer Antischweißzusammensetzung nach einem der Ansprüche 1 bis 16 darauf.

**Revendications**

1. Une composition antiperspirante appropriée pour l'application locale sur la peau de l'être humain, comprenant une quantité efficace d'un sel de titane d'un hydroxyacide carboxylique aliphatique présentant une longueur de chaîne comprise entre $C_2$ et $C_{10}$, la composition présentant un pH en solution aqueuse supérieur à 5.

2. Une composition antiperspirante selon la Revendication 1, la composition comprenant 0,1 à 50 % en masse du sel de titane.

3. Une composition antiperspirante selon la Revendication 2, la composition comprenant 10 à 40 % en masse du sel de titane.

4. Une composition antiperspirante selon la Revendication 2, la composition comprenant 1 à 10 % en masse du sel de titane.

5. Une composition antiperspirante selon l'une quelconque des Revendications précédentes, le pH de la composition en solution aqueuse étant supérieur à 5,5.

6. Une composition antiperspirante selon la Revendication 5, le pH de la composition en solution aqueuse étant compris dans la gamme allant de 6 à 10.

7. Une composition antiperspirante selon la Revendication 6, le pH de la composition en solution aqueuse étant compris dans la gamme allant de 6 à 7.

8. Une composition antiperspirante selon l'une quelconque des Revendications précédentes, le sel de titane étant un sel de mono- ou di-acide de citrate, de gluconate, de glycolate ou de lactate.

9. Une composition antiperspirante selon la Revendication 8, la matière active antiperspirante étant un sel de lactate d'ammonium et de titane.

10. Une composition antiperspirante selon l'une quelconque des Revendications précédentes, qui est substantiellement anhydre et comprend en outre de l'éthanol, de l'isopropanol ou un mélange de ceux-ci.

11. Une composition antiperspirante selon la Revendication 10, l'isopropanol, l'éthanol ou un mélange de ceux-ci étant présent dans la composition selon une proportion comprise entre 50 et 80 % en masse.

12. Une composition antiperspirante selon l'une quelconque des Revendications précédentes, comprenant en outre une argile hydrophobe.

13. Une composition antiperspirante selon la Revendication 12, l'argile hydrophobe étant présente dans la composition selon une proportion comprise entre 5 et 20 % en masse.

14. Une composition antiperspirante selon l'une quelconque des Revendications précédentes, comprenant en outre une silicone volatile ou non volatile.

15. Une composition antiperspirante selon l'une quelconque des Revendications précédentes, comprenant en outre 0,1 à 2 % en masse d'un agent tensioactif.

16. Une composition antiperspirante selon l'une quelconque des Revendications précédentes, qui revêt la forme d'un stick solide, d'une lotion, d'une crème ou d'un spray aérosol.

17. Une méthode non thérapeutique de prévention ou de limitation de la transpiration de la peau de l'être humain, comprenant l'étape consistant à appliquer localement sur celle-ci une composition antiperspirante selon l'une quelconque des Revendications 1 à 16.

Fig.1

Fig. 2